# EUROPÄISCHE PATENTANMELDUNG

(11) **EP 3 964 653 A1**
(43) Veröffentlichungstag der Anmeldung: **09.03.2022**
(21) Anmeldenummer: 20195136.5
(22) Anmeldetag: 08.09.2020
(51) Int. Cl.: E03C 1/05, G06K 9/00, G08B 21/24, E03C 1/046

(54) **HYGIENESYSTEM UND VERFAHREN ZUM BETREIBEN EINES HYGIENESYSTEMS**

(71) Anmelder: Ideal Standard International NV, 1935 Zaventem (BE)
(72) Erfinder: Depiere, Bert, 3150 Haacht (BE); Donell, Ralf, 54497 Morbach (DE); Mettler, Rainer, 54518 Altrich (DE)
(74) Vertreter: Feucker, Max Martin

(57) **Zusammenfassung**

Die vorliegende Erfindung betrifft ein Hygienesystem, umfassend
- mindestens eine Sanitärarmatur (1), wobei die Sanitärarmatur mindestens einen Fluidauslass (2.1, 2.2) zur Ausgabe mindestens eines Fluids aufweist,
- mindestens eine Kamera (3) zur Aufnahme der Hände (6) eines Benutzers,
- mindestens eine optische Anzeige (4) und
- eine Steuerung (5), wobei die Steuerung (5) mit der Kamera (3) und mit der optischen Anzeige (4) verbunden ist.

## Beschreibung

Die vorliegende Erfindung betrifft ein Hygienesystem, umfassend eine Sanitärarmatur, wobei die Sanitärarmatur mindestens einen Fluidauslass zur Ausgabe mindestens eines Fluids (wie Wasser, Seife und/oder Desinfektionsmittel) aufweist, eine Kamera zur Aufnahme der Hände eines Benutzers, eine optische Anzeige und eine Steuerung, wobei die Steuerung mit der Kamera und mit der Anzeige datentechnisch verbunden ist. Die Erfindung betrifft zudem ein Verfahren zum Betreiben eines Hygienesystems, welches die Verfahrensschritte des Aufnehmens der Hände eines Benutzers bei einem Reinigungsvorgang und der prozessorgestützten Auswertung der Aufnahme umfasst.

Für eine hygienegerechte Reinigung der Hände beziehungsweise einer ausreichend zuverlässigen Entfernung von Viren und Bakterien von den Händen ist es erforderlich, dass alle Handbereiche (z.B. Handballen, Handinnenfläche, Finger, Fingerzwischenräume, Handaußenseite, Fingernägel) ausreichend lange und mit dem dafür erforderlichen mechanischen Aneinanderreiben der Hände unter Verwendung von Seife oder einem Desinfektionsmittel gereinigt werden. Aus DE 60 308 838 T2 und EP 2 015 665 B1 ist es daher bekannt, den Waschvorgang mittels einer Kamera aufzunehmen und die Aufnahmen prozessorgesteuert in der Hinsicht auszuwerten, ob eine ausreichende Reinigung stattgefunden hat. Nach der Auswertung wird dem Benutzer allerdings lediglich mitgeteilt, ob der Reinigungsvorgang vorschriftsmäßig durchgeführt wurde oder nicht. Wenn ein Benutzer nach seiner Meinung ausreichend seine Hände gereinigt hat, so reicht eine solche allgemeine Rückmeldung nicht immer aus, um den Benutzer zu motivieren, seine Hände nochmals zu reinigen. Dies ist dann insbesondere bei Benutzern aus dem Bereich des Pflegepersonals mit der Gefahr verbunden, dass sich der Benutzer selbst oder eine von dem Benutzer betreute Person über die unter Umständen noch an den Händen befindlichen Bakterien/Viren infiziert.

Aufgabe der vorliegenden Erfindung ist es daher, die mit Bezug zum Stand der Technik geschilderten Nachteile zu beseitigen und insbesondere ein Hygienesystem und ein Verfahren zum Betreiben eines solchen Hygienesystems anzugeben, mit welchen die Handhygiene insbesondere von Benutzern im Pflegebereich oder anderen kritischen Hygienebereichen verbessert werden kann.

Gelöst wird die Aufgabe durch ein Hygienesystem und ein Verfahren mit den Merkmalen des jeweiligen unabhängigen Anspruchs. Vorteilhafte Weiterbildungen des Hygienesystems und des Verfahrens sind in den abhängigen Ansprüchen und in der Beschreibung angegeben, wobei einzelne Merkmale der vorteilhaften Weiterbildungen in technisch sinnvoller Weise miteinander kombinierbar sind. Insbesondere sind die mit Bezug zum Hygienesystem offenbarte Merkmale auf das Verfahren anwendbar und umgekehrt.

Gelöst wird die Aufgabe insbesondere durch ein Hygienesystem mit den eingangs genannten Merkmalen, bei dem die Steuerung dazu eingerichtet ist, die Handbereiche der Hände eines Nutzers festzustellen, die gereinigt wurden, und bei dem die Steuerung und die optische Anzeige dazu eingerichtet sind, wenigstens einen Handbereich auf der optischen Anzeige anzuzeigen, wenn festgestellt wird, dass der entsprechende Handbereich nicht oder nicht ausreichend gereinigt wurde.

Gelöst wird die Aufgabe insbesondere auch durch ein Verfahren zum Betreiben eines Hygienesystems mit mindestens einem Auslass für ein Fluid, mit einer Kamera und mit einer optischen Anzeige, umfassend zumindest die folgenden Schritte:
- Aufnehmen der Hände eines Benutzers bei einem Reinigungsvorgang,
- Prozessorgestützte Auswertung der Aufnahmen, wobei festgestellt wird, welche Handbereiche der Hände gereinigt wurden, und
- Anzeigen wenigstens eines Handbereichs auf der optischen Anzeige, wenn festgestellt wird, dass der entsprechende Handbereich nicht oder nicht ausreichend gereinigt wurde.

Die Erfindung schlägt also sinngemäß vor, dass dem Benutzer nicht nur angezeigt wird, dass die Hände nicht ausreichend gereinigt wurden, sondern auch welcher Bereich seiner Hände nicht ausreichend gereinigt wurde. Somit weiß der Benutzer konkret, welcher Handbereich in einem ergänzenden Reinigungsvorgang zu reinigen ist, sodass davon ausgegangen werden kann, dass Nutzer häufiger einen zusätzlichen Reinigungsvorgang vornehmen. Somit wird die Handhygiene wesentlich verbessert. Dementsprechend weist die optische Anzeige bevorzugt einen insbesondere zweidimensionalen Bildschirm auf, auf dem ein Handbereich, eine Hand oder beide Hände zumindest stilisiert dargestellt werden kann, wobei durch eine Hervorhebung (beispielsweise Umrandung, farblicher Kontrast oder Ähnliches) hervorgehoben wird, welcher konkrete Handbereich (beispielsweise ein konkreter Finger oder konkrete Finger, die Handinnenfläche, ein konkreter Bereich der Handinnenfläche oder Handaußenfläche) nicht ausreichend gereinigt wurde. Insbesondere wird durch die Darstellung auch hervorgehoben, ob ein Handbereich der linken und/oder der rechten Hand nicht ausreichend gereinigt wurde.

Die optische Anzeige ist insbesondere so angeordnet, dass ein Benutzer nach einem Reinigungsvorgang auf die optische Anzeige aufmerksam wird. So kann beispielsweise ein eigenständiger Bildschirm an einer Wand des Waschraums angebracht sein oder in einem Spiegel integriert sein. Bevorzugt ist jedoch, dass die optische Anzeige in einem Gehäuse der Sanitärarmatur integriert ist. Dies vereinfacht zum einen den Montageaufwand des Hygienesystems. Zudem kann es zu keiner Verwechslung zwischen der Anzeige und der zugehörigen Wascharmatur kommen, wenn in einem Waschraum mehrere Sanitärarmaturen aufgestellt sind.

Von der Erfindung in ihrem allgemeinsten Grundgedanken wäre aber auch umfasst, dass der Benutzer nach dem Reinigungsvorgang eine personalisierte Nachricht auf ein (mobiles) Endgerät erhält, auf dessen Bildschirm der nicht gereinigte Handbereich angezeigt wird. Hierzu wäre eine noch weiter unten erläuterte Identifizierung des Benutzers erforderlich sowie eine zentrale Ausbildung zumindest eines Teils der Steuerung, wie sie auch nachfolgend noch beschrieben wird.

Die mindestens eine Kamera ist so ausgerichtet, dass die Hände des Benutzers bei einem Reinigungsvorgang (beispielsweise Desinfektionsvorgang oder Waschvorgang) aufgenommen werden. Die Kamera kann hierzu prinzipiell benachbart zu der Sanitärarmatur angeordnet sein. Bevorzugt ist jedoch, dass die Kamera in einem Gehäuse der Sanitärarmatur integriert ist und insbesondere auf das Waschbecken gerichtet ist. Somit ist zum einen der Installationsaufwand verringert und zum anderen eine kompakte Einheit gegeben. In einer bevorzugten Ausführungsform ist die Kamera insbesondere so angeordnet, dass ein Gesicht eines Benutzers nicht aufgenommen wird, sodass auch keine Aufnahmen von dem Gesicht eines Benutzers gespeichert werden könnten.

Die Sanitärarmatur ist insbesondere eine Badarmatur mit mindestens einem Fluidauslass für Wasser. Ein weiterer Fluidauslass für Seife kann in dasselbe Gehäuse der Sanitärarmatur integriert sein. Alternativ könnte ein Seifenauslass in einem benachbarten Gehäuse, beispielsweise eines Seifenspenders, integriert sein. Die Sanitärarmatur kann aber auch als Desinfektionsspender zur Ausgabe von Desinfektionsmittel ausgebildet sein.

Insbesondere umfasst die Sanitärarmatur mindestens einen weiteren, zweiten Auslass zur Ausgabe eines zweiten, sich von dem ersten Fluid unterscheidenden Fluid, wobei sowohl die Ausgabe des ersten Fluids aus dem ersten Auslass als auch die Ausgabe des zweiten Fluids aus dem zweiten Auslass durch die Steuerung gesteuert werden kann. Aus dem ersten Auslass wird insbesondere Wasser als erstes Fluid ausgegeben und aus dem zweiten Auslass wird insbesondere Seife als zweites Fluid ausgegeben. Die Steuerung kann in diesem Zusammenhang nicht nur dazu eingerichtet sein, den Waschvorgang aufzunehmen und das Waschergebnis anzuzeigen, sondern auch einen vorgegebenen Ablauf von Ausgabe der Fluide (insbesondere deren Reihenfolge und Länge) nach Detektion eines Benutzers zu steuern.

Die Steuerung kann insbesondere auch so eingerichtet sein, dass ein neuer Reinigungsvorgang (beispielsweise durch die Ausgabe von Wasser, Seife und/oder Desinfektionsmittel) eingeleitet wird, wenn festgestellt wurde, dass ein Handbereich eines Benutzers nicht ausreichend gereinigt wurde. Somit kann der Benutzer gegebenenfalls motiviert werden, den nicht gereinigten Handbereich zu reinigen, ohne selbst den Reinigungsvorgang auslösen zu müssen.

Die Steuerung umfasst einen Algorithmus zur Auswertung der Handbewegungen und/oder der Verteilung des Reinigungsmittels (beispielsweise Seife oder Desinfektionsmittel) während des Reinigungsvorgangs. Die dabei festgestellten Bewegungen der Hände des Benutzers und die Verteilung des Reinigungsmittels während der Bewegungen werden mit vorgegebenen Kriterien verglichen. Alternativ oder zusätzlich kann die Steuerung auch eine künstliche Intelligenz aufweisen, die zur Erkennung eines ausreichenden Reinigungsvorgangs angelernt ist und die dazu angelernt ist, die nicht gereinigten Handbereiche eines Benutzers festzustellen.

Insbesondere kann auch ausgewertet und als Ergebnis auf der optischen Anzeige angezeigt werden, ob ausreichend Wasser, Seife und/oder Desinfektionsmittel bei dem Reinigungsvorgang verwendet wurde.

Wenn die Steuerung feststellt, dass alle Handbereiche des Benutzers ausreichend gereinigt wurden, so kann auch ein solches perfektes Reinigungsergebnis durch geeignete Symbole auf der optischen Anzeige angezeigt werden.

In einer Ausführungsform kann vorgesehen sein, dass die vollständige Auswertung der Aufnahmen lokal mit einer prozessorgesteuerten Einheit erfolgt. In diesem Fall ist die Steuerung jeder Sanitärarmatur also autark und bedarf keiner weiteren datentechnischen Anbindung an das Internet und insbesondere an ein zentrales Rechenzentrum ("Cloud").

In einer weiteren Ausführungsform kann die lokale Einheit der Steuerung ein Kommunikationsmodul umfassen, mittels dem Daten zu einer zweiten, insbesondere über das Internet verbundenen Einheit (Rechenzentrum) gesendet werden können. Die zweite Einheit der Steuerung ist also von der Sanitärarmatur entfernt angeordnet, während die erste mit der Kamera verbundene Einheit lokal, beispielsweise benachbart zu oder in der Sanitärarmatur angeordnet ist.

Bei Ausbildung eines Kommunikationsmoduls kann nun vorgesehen sein, dass das Ergebnis einer Auswertung durch die erste Einheit an die zweite Einheit übermittelt wird, sodass die zweite Einheit eine Analyse der übermittelten Daten und insbesondere eine Analyse der von der ersten Einheit ermittelten Ergebnisse vornehmen kann. In diesem Fall können aber auch die Ergebnisse der lokalen Auswertung zur weiteren Verbesserung des Algorithmus (Stichwort "künstliche Intelligenz") an die zweite Einheit übermittelt werden.

Alternativ kann aber auch vorgesehen sein, dass die von der Kamera aufgenommenen Daten von der ersten Einheit quasi in Echtzeit an die zweite Einheit übermittelt werden, in welchem Fall eine Auswertung der Aufnahmen in der zweiten Einheit, also insbesondere in einem Rechenzentrum (Stichwort "Cloudcomputing") erfolgt. Das Ergebnis dieser Auswertung wird dann wiederum an die erste Einheit übermittelt, die das Ergebnis einem Benutzer auf einer mit der Sanitärarmatur assoziierten optischen Anzeige anzeigt. Eine solche Variante hat den Vorteil, dass die lokale, mit der Sanitärarmatur verbundene erste Einheit keine große Rechenleistung benötigt, da die Auswertung in der zweiten Einheit erfolgt.

Insbesondere wenn eine im Internet angeordnete zweite Einheit vorgesehen ist, so kann auch eine Benutzerverwaltung vorgesehen sein, sodass ein vor oder während des Reinigungsvorgangs identifizierter Benutzer eine personalisierte Nachricht mit der Anzeige des Reinigungsergebnisses erhält.

Prinzipiell ist es aber möglich, wenn die Auswertung des Reinigungsvorgangs anonym erfolgt und der (anonyme) Benutzer den nicht ausreichend gereinigten Handbereich angezeigt bekommt.

In manchen Anwendungsfällen könnte es hingegen wünschenswert sein, wenn das Reinigungsergebnis einem konkreten Benutzer zugeordnet wird. Zudem ist es in einigen Anwendungsfällen von Vorteil, wenn einem Benutzer zwar personalisiert aber anonym das Reinigungsergebnis übermittelt wird. In beiden Fällen ist es also erforderlich, dass der Benutzer (anonym oder nicht anonym) identifiziert wird. In diesem Fall umfasst das Hygienesystem und insbesondere die Sanitärarmatur eine Identifizierungseinrichtung zur Identifikation des Benutzers. Die Identifizierungseinrichtung kann insbesondere einen Fingerabdrucksensor oder einen anderen Sensor zur Erkennung eines körperlichen Merkmals des Benutzers umfassen. Alternativ oder zusätzlich kann eine Einheit zur Erkennung einer vorgefertigten Identifizierung wie Barcode, RFID Chip oder Smartphone vorgesehen sein. Wenn eine zweite Einheit vorgesehen ist, so kann das Hygienesystem eine Vielzahl gegebenenfalls auch an unterschiedlichen Orten installierte Sanitärarmaturen aufweisen. Aufgrund der Verbindung mit einer zentralen zweiten Einheit kann eine sanitärarmaturbezogene, eine waschraumbezogene und/oder eine globale Analyse der ausgewerteten Daten angefertigt werden. Aus einer solchen Analyse können dann Schlüsse für verbesserte Maßnahmen zur Handhygiene gezogen werden.

So kann insbesondere mittels der zweiten Einheit erfasst werden, wann und wie lange die Hände (im Durchschnitt) gereinigt werden, wie häufig bei mehreren Reinigungsvorgängen (insbesondere mit unterschiedlichen Reinigungsmitteln) insbesondere bei aufeinanderfolgenden Reinigungsvorgängen alle Handbereiche gereinigt werden. Es kann also festgestellt werden, ob es eine unterschiedliche Erfolgsrate bei der Verwendung unterschiedlicher Reinigungsmittel gibt beziehungsweise wie häufig es erforderlich ist, mehrere nacheinander erfolgende Reinigungsvorgänge zur vollständigen Reinigung der Hände auszuführen. Zudem kann auch analysiert werden, welche Anzahl an Reinigungszyklen ein Benutzer bzw. alle Benutzer im Durschnitt ausführt/ausführen, bis alle Handbereiche gereinigt sind.

Zudem kann analysiert werden, wie viele Reinigungsvorgänge überhaupt mittels einer Sanitärarmatur ausgeführt werden.

Wenn auch ein Sensor zur Erfassung der in einen Waschraum eintretenden und austretenden Personen vorgesehen ist, so kann auch die Anzahl der in einem vorgegebenen Zeitraum (täglich, wöchentlich) in dem Waschraum befindlichen Personen erfasst werden. In diesem Fall kann analysiert werden, ob jede Person im Waschraum einen Reinigungsvorgang durchführt oder wie hoch deren Anteil ist.

Die gegebenenfalls anonym gesammelten Daten für einen Waschraum, ein Gebäude, ein Unternehmen oder global können somit zu einfachen Auswertung bereitstehen. Es ist aber auch möglich, dass für genau einen Benutzer eine Statistik angelegt wird und diesem Benutzer zu seiner Information bereitgestellt wird. In hygienekritischen Bereichen (wie beispielsweise in Krankenhäusern) kann aber auch der Reinigungsvorgang personalisiert für alle Benutzer, welche der Erfassung zugestimmt haben, erfasst und für eine Verbesserung der Handhygiene ausgewertet werden.

Die Erfindung sowie das technische Umfeld werden im Folgenden anhand der Figuren beispielhaft erläutert. Es zeigen schematisch
- Figur 1:: ein Hygienesystem mit einem Sanitärarmatur,
- Figur 2:: ein Schaltbild des Hygienesystems,
- Figur 3:: ein Schaltbild einer zweiten Ausführungsform eines Hygienesystems,
- Figur 4a bis 4c:: Darstellungen auf einer Anzeige des Hygienesystems und
- Figur 5:: einen Ablaufplan beim Betreiben des Hygienesystems.

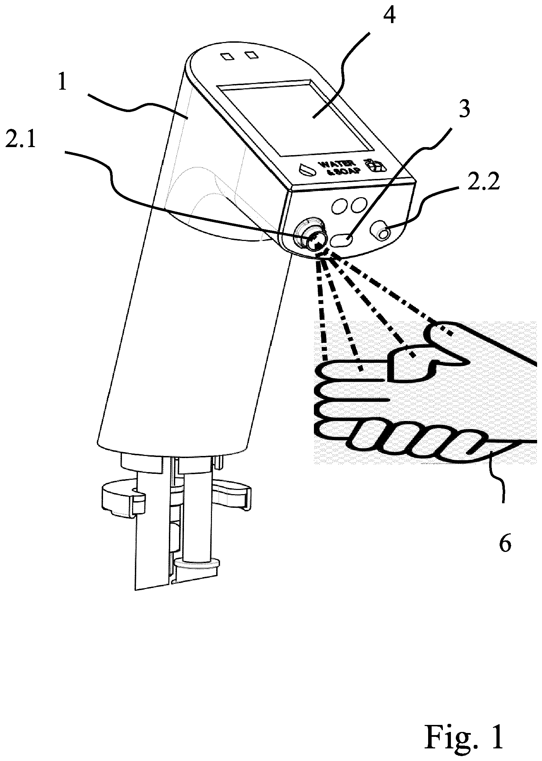

Die in Figur 1 dargestellte Sanitärarmatur 1 umfasst einen ersten Fluidauslass 2.1 für Wasser und einen zweiten Fluidauslass 2.2 für Seife. In der Sanitärarmatur 1 ist zudem eine Kamera 3 integriert, die die Hände 6 eines Benutzers während eines Waschvorgangs aufnimmt. In der Sanitärarmatur 1 ist eine in Figur 1 nicht dargestellte Steuerung integriert, die mit der Kamera 3 und einer optischen Anzeige 4 verbunden ist. Die optische Anzeige 4 ist dabei so ausgebildet, dass auf dieser zumindest stilisiert Handbereiche oder Hände dargestellt werden können.

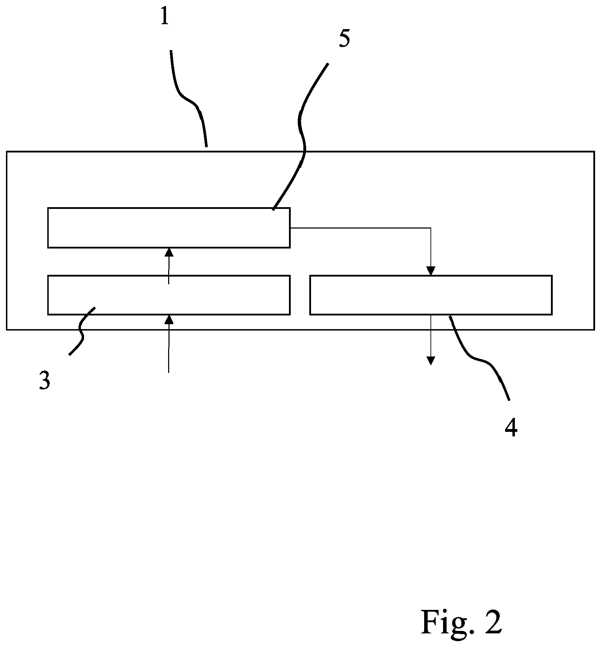

Wie aus der Figur 2 hervorgeht, wird mittels der Kamera 3 der Waschvorgang der Hände 6 des Benutzers aufgezeichnet, wobei die Aufnahmen der Kamera 3 in der Steuerung 5 ausgewertet werden, wobei die Steuerung 5 daraufhin eine Darstellung auf der optischen Anzeige 4 veranlasst. Bei dem in Figur 2 dargestellten Ausführungsbeispiel erfolgt die gesamte Auswertung in der in der Sanitärarmatur 1 angeordneten Steuerung 5.

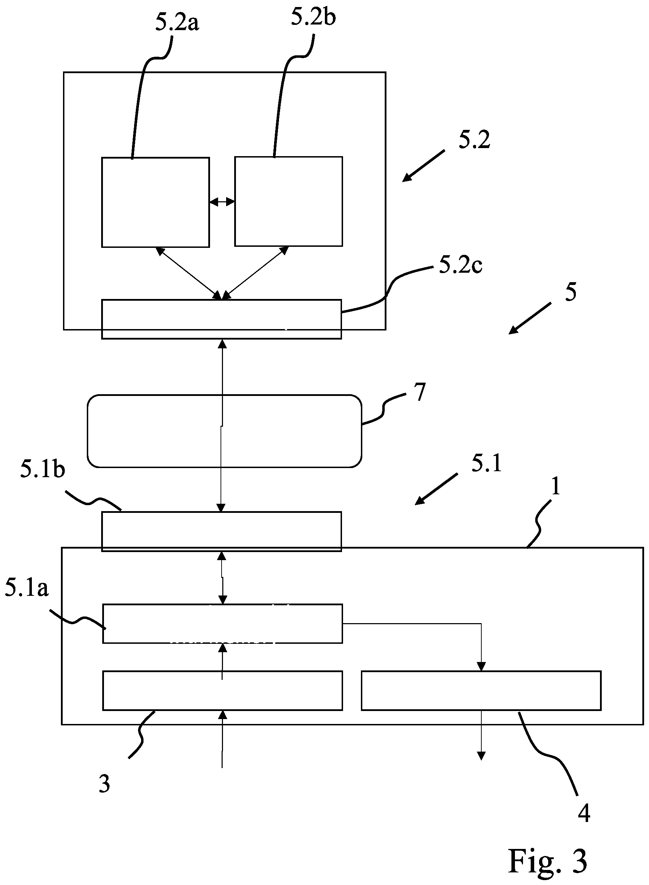

Wie aus der Ausführungsform der Figur 3 hervorgeht, kann die Steuerung 5 aber auch mehrere Module/Einheiten umfassen. So ist die in der Sanitärarmatur 1 integrierte Kamera 3 mit einem Datenverarbeitungsmodul 5.1a einer ersten Einheit 5.1 verbunden. Die mit der Sanitärarmatur 1 assoziierte erste Einheit 5.1 umfasst zudem ein Kommunikationsmodul 5.1b. Die Steuerung 5 umfasst zudem eine zweite Einheit 5.2 die beispielsweise durch ein Rechenzentrum gebildet ist. Die zweite Einheit 5.2 umfasst ein Kommunikationsmodul 5.2c, eine Datenverarbeitungseinheit 5.2a und einen Speicher 5.2b. Die Kommunikationsmodule 5.1b und 5.2c können entweder direkt oder über ein Gateway 7 miteinander Daten austauschen.

Die Steuerung 5 ist in jedem Fall dahingehend eingerichtet, anhand der von der Kamera 3 aufgenommenen Aufnahmen zu erkennen, ob ein Benutzer alle Handbereiche 6a, 6b ausreichend gereinigt hat. Wird festgestellt, dass einer oder mehrere Handbereiche der Hände 6 nicht ausreichend gereinigt wurde(n), so wird der nicht gereinigte Bereich auf der optischen Anzeige 4 angezeigt. Der Benutzer weiß somit sofort, welcher Bereich seiner Hände bei einem weiteren Reinigungsvorgang gereinigt werden muss.

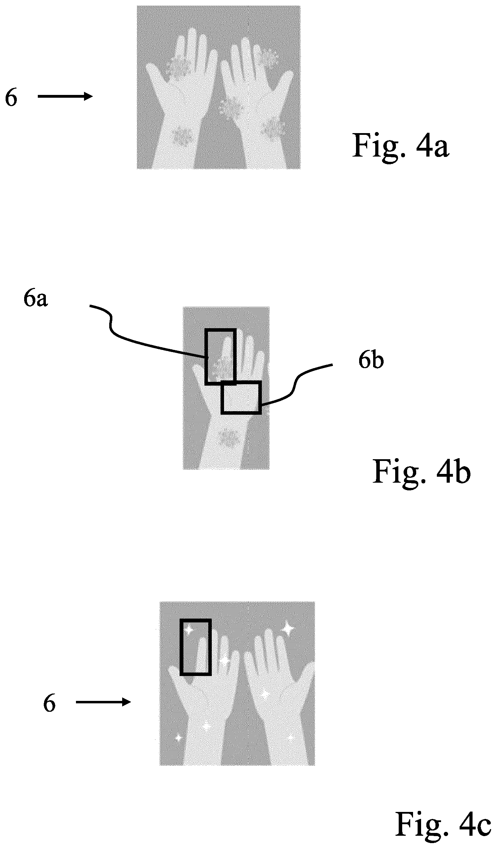

Wie in Figur 4a dargestellt, kann dem Benutzer beispielsweise angezeigt werden, dass gar kein Bereich seiner Hände ausreichend gereinigt wurde.

Gemäß der Darstellung in Figur 4b wird dem Benutzer durch eine Hervorhebung angezeigt, welcher konkrete Handbereich 6a, 6b nicht ausreichend gereinigt wurde. Wird hingegen festgestellt, dass die Hände 6 ausreichend gereinigt wurden, so kann dies, wie in Figur 4c gezeigt, auf der optischen Anzeige 4 dargestellt werden.

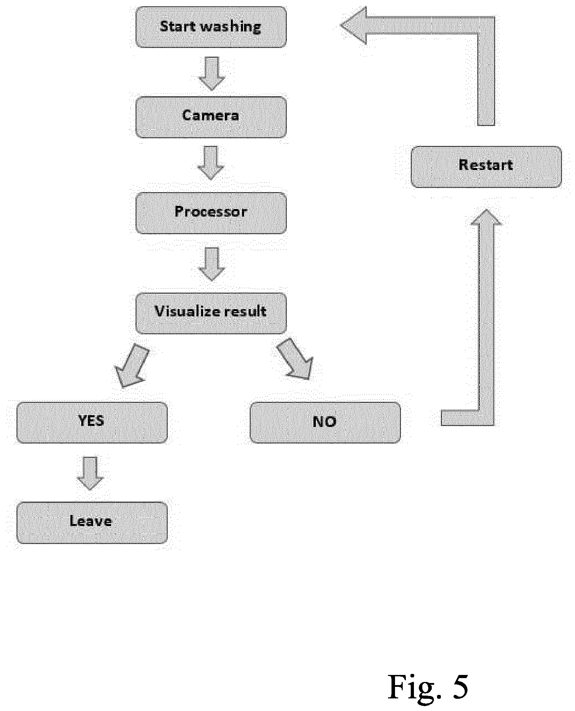

In Figur 5 ist der Ablauf eines solchen Waschvorgangs dargestellt. So wird nach dem anfänglichen Start mit der Kamera 3 der Waschvorgang aufgenommen, wobei die aufgenommenen Bilder prozessorgestützt ausgewertet werden, und das Ergebnis der Auswertung optisch dargestellt wird, indem der nicht ausreichend gereinigte Handbereich 6a, 6b angezeigt wird. Es kann zudem vorgesehen sein, dass die Ausgabe des Reinigungsmittels oder von Wasser automatisch neu gestartet wird, wenn ein nicht ausreichend gereinigter Handbereich 6a, 6b festgestellt wurde. Wenn hingegen schon beim ersten Reinigungsvorgang ein perfektes Reinigungsergebnis erzielt wird, so wird auch dies angezeigt, und der Benutzer kann den Waschraum verlassen.

Gemäß der in Figur 2 dargestellten Ausführungsform erfolgt die komplette Auswertung der von der Kamera 3 aufgenommenen Bilder in der in der Sanitärarmatur 1 integrierten Steuerung 5.

Bei der in Figur 3 dargestellten Ausführungsform kann hingegen entweder die komplette Auswertung in dem Datenverarbeitungsmodul 5.1a erfolgen und das Ergebnis der Auswertung mittels des Kommunikationsmoduls 5.1b an die zweiten Einheit 5.2 übertragen werden. Alternativ können die von der Kamera 3 aufgenommen Bilder über das Datenverarbeitungsmodul 5.1a und das Kommunikationsmodul 5.1b quasi in Echtzeit an die zweite Einheit 5.2 übertragen werden, in welcher die Auswertung der von der Kamera 3 aufgenommen Bilder erfolgt. Das Ergebnis der Auswertung wird dann wiederum in dem Speicher 5.2b abgelegt und über die Kommunikationsmodule 5.2c und 5.1b an die erste Einheit 5.1 übertragen, sodass die nicht ausreichend gereinigten Handbereiche 6a, 6b auf der optischen Anzeige 4 angezeigt werden können.

Die Anbindung der Sanitärarmatur 1 an eine zweite, sich im Internet befindliche Einheit 5.2 hat insbesondere den Vorteil, dass die ausgewerteten Ergebnisse, wie in der allgemeinen Beschreibung erläutert, analysiert und zur Verbesserung des Betriebs der Waschräume eingesetzt werden können.

### Bezugszeichen

- 1: Sanitärarmatur
- 2.1, 2.2: Fluidauslass
- 3: Kamera
- 4: optische Anzeige
- 5: Steuerung
- 5.1: erste Einheit
- 5.1a: Datenverarbeitungsmodul
- 5.1b: Kommunikationsmodul
- 5.2: zweite Einheit
- 5.2a: Datenverarbeitungseinheit
- 5.2b: Speicher
- 5.2c: Kommunikationsmodul
- 6: Hände
- 6a, 6b: Handbereich
- 7: Gateway

## Patentansprüche

1. Hygienesystem, umfassend
- mindestens eine Sanitärarmatur (1), wobei die Sanitärarmatur mindestens einen Fluidauslass (2.1, 2.2) zur Ausgabe mindestens eines Fluids aufweist,
- mindestens eine Kamera (3) zur Aufnahme der Hände (6) eines Benutzers,
- mindestens eine optische Anzeige (4) und
- eine Steuerung (5), wobei die Steuerung (5) mit der Kamera (3) und mit der optischen Anzeige (4) verbunden ist,
**dadurch gekennzeichnet, dass**
- die Steuerung (5) dazu eingerichtet ist, die Handbereiche (6a, 6b) der Hände (6) eines Benutzers festzustellen, die gereinigt wurden, und
- die Steuerung (5) und die optische Anzeige (4) dazu eingerichtet sind, wenigstens einen Handbereich (6a) auf der optischen Anzeige (4) anzuzeigen, wenn festgestellt wird, dass der entsprechende Handbereich (6a) nicht oder nicht ausreichend gereinigt wurde.

2. Hygienesystem nach Anspruch 1, wobei die Steuerung (5) dazu eingerichtet ist, ein perfektes Reinigungsergebnis auf der optischen Anzeige (4) anzuzeigen, wenn alle Handbereiche (6a, 6b) ausreichend gereinigt wurden.

3. Hygienesystem nach einem der vorhergehenden Ansprüche, wobei die Sanitärarmatur (1) eine Identifizierungseinrichtung zur Identifikation des Benutzers umfasst.

4. Hygienesystem nach Anspruch 3, wobei die
Identifizierungseinrichtung eines der folgenden Elemente umfasst:
- Fingerabdrucksensor oder Sensor zur Erkennung des Benutzers an einer körperlichen Eigenschaft,
- Einheit zur Erkennung einer vorgefertigten Identifizierung wie Barcode, RFID Chip oder Smartphone,
wobei insbesondere eine personalisierte Rückmeldung an den Benutzer erfolgt.

5. Hygienesystem nach einem der vorhergehenden Ansprüche, wobei die Sanitärarmatur (1) mindestens einen weiteren, zweiten Auslass (2.2) zur Ausgabe eines zweiten Fluids aufweist und die Ausgabe des ersten Fluids aus dem ersten Auslass (2.1) und des zweiten Fluids aus dem zweiten Auslass (2.2) durch die Steuerung (5) gesteuert wird.

6. Hygienesystem nach einem der vorhergehenden Ansprüche, wobei die Steuerung (5) eine mit der Kamera (3) verbundene erste Einheit (5.1) und eine von der Sanitärarmatur (1) entfernt angeordnete zweite Einheit (5.2) aufweist.

7. Hygienesystem nach Anspruch 6, wobei die erste Einheit (5.1) ein mit der Kamera (3) verbundenes Datenverarbeitungsmodul (5.1a) und ein Kommunikationsmodul (5.1b) zur Kommunikation mit der zweiten Einheit (5.2) aufweist.

8. Hygienesystem nach Anspruch 6 oder 7, **dadurch gekennzeichnet, dass** in Analysen der zweiten Einheit (5.2) mindestens eine der folgenden Auswertungen in einer Datenverarbeitungseinheit (5.2a) vorgenommen wird und/oder in einem Speicher (5.2b) gespeichert wird:
- wann und wie lange die Hände (6) gereinigt wurden,
- wie häufig bei mehreren Reinigungsvorgängen alle Handbereiche (6a ,6b) gereinigt wurden,
- Anzahl der Reinigungszyklen eines Benutzers, bis alle Handbereiche (6a ,6b) gereinigt sind,
- Anzahl der durchgeführten Reinigungsvorgänge,
- Anzahl der Personen im Waschraum,
- ob der Reinigungsvorgang von jeder Person im Waschraum durchgeführt wurde.

9. Hygienesystem nach einem der vorhergehenden Ansprüche, wobei die Kamera (3), die optische Anzeige (4) und zumindest eine erste Einheit (5.1) der Steuerung (5) in einem Gehäuse der Sanitärarmatur (1) angeordnet sind.

10. Verfahren zum Betreiben eines Hygienesystems mit mindestens einem Auslass (2.1,2.2) für ein Fluid, mit einer Kamera (3) und mit einer optischen Anzeige (4), insbesondere zum Betreiben eines Hygienesystems nach einem der vorhergehenden Ansprüche, umfassend zumindest die folgenden Schritte:
- Aufnehmen der Hände (6) eines Benutzers bei einem Reinigungsvorgang,
- Prozessorgestützte Auswertung der Aufnahmen, wobei festgestellt wird, welche Handbereiche (6a, 6b) der Hände (6) gereinigt wurden,
- Anzeigen wenigstens eines Handbereichs (6a) auf der optischen Anzeige (4), wenn festgestellt wird, dass der entsprechende Handbereich (6a) nicht oder nicht ausreichend gereinigt wurde.

11. Verfahren nach Anspruch 10, wobei ein perfektes Reinigungsergebnis auf der optischen Anzeige (4) angezeigt wird, wenn alle Handbereiche (6a, 6b) ausreichend gereinigt wurden.

12. Verfahren nach einem der Ansprüche 10 oder 11, wobei vor der Reinigung die Identität des Benutzers erfasst wird.

13. Verfahren nach Anspruch 12, wobei der Benutzer nach dem Reinigungsvorgang eine personalisierte Rückmeldung erhält.

14. Verfahren nach einem der Ansprüche 10 bis 13, wobei
- eine erste Verarbeitung der Aufnahmen auf einem mit der Kamera verbundenen Datenverarbeitungsmodul (5.1a) einer ersten Einheit (5.1) erfolgt,
- die verarbeiteten Daten an eine zweite Einheit (5.2) übertragen werden,
- die zweite Einheit (5.2) eine Auswertung der bearbeiteten Daten vornimmt und
- ein Ergebnis der Auswertung an die erste Einheit (5.1) übertragen wird.

15. Verfahren nach einem Anspruch 9 bis 14, **dadurch gekennzeichnet, dass** mindestens eine der folgenden Analysen vorgenommen wird und gespeichert wird:
- wann und wie lange die Hände (6) gereinigt wurden,
- wie häufig bei mehreren Reinigungsvorgängen alle Handbereiche (6a, 6b) gereinigt wurden,
- Anzahl der Reinigungszyklen eines Benutzers, bis alle Handbereiche (6a, 6b) gereinigt sind,
- Anzahl der durchgeführten Reinigungsvorgänge,
- Anzahl der Personen im Waschraum,
- ob der Reinigungsvorgang von jeder Person im Waschraum durchgeführt wurde.
